# EUROPEAN PATENT SPECIFICATION

(11) **EP 2 007 376 B1**
(45) Date of publication and mention of the grant of the patent: **08.05.2013**
(21) Application number: 07713339.5
(22) Date of filing: 13.03.2007
(51) Int. Cl.: A61K 31/352, A61K 31/35

(54) **THERAPEUTIC USES OF CANNABIDIOL COMPOUNDS**
THERAPEUTISCHE VERWENDUNGEN VON CANNABIDIOL-VERBINDUNGEN
UTILISATIONS THÉRAPEUTIQUES DE COMPOSÉS DE CANNABIDIOL

(30) Priority: 13.03.2006 US 781345 P
(43) Date of publication of application: 31.12.2008
(73) Proprietor: HADASIT MEDICAL RESEARCH SERVICES AND DEVELOPMENT LTD., 91120 Jerusalem (IL); Yissum Research Development Company of the Hebrew University of Jerusalem, Jerusalem 91390 (IL)
(72) Inventor: WEISS, Lola, 95743 Jerusalem (IL); MEINER, Vardiella, 96926 Jerusalem (IL); LEITERSDORF, Eran, 90805 Mevaseret Zion (IL); SLAVIN, Shimon, 95744 Jerusalem (IL); MECHOULAM, Raphael, 92581 Jerusalem (IL); GALLILY, Ruth, 93706 Jerusalem (IL); LOTAN, Chaim, 96263 Jerusalem (IL); DURST, Ronen, 83006 Mevaseret Zion (IL); PUGATSCH, Thea, 98330 Maale Adumim (IL)
(74) Representative: Fleuchaus, Andrea
(86) International application number: PCT/IL2007/000319
(87) International publication number: WO 2007/105210

(56) References cited:
- WO-A-99/53917
- WO-A2-01/95899
- US-B1- 6 563 009

## Description

### FIELD OF THE INVENTION

This invention relates to the use of cannabidiol compounds in therapy.

### PRIOR ART

The following is a list of prior art which are considered to be pertinent for describing the state of the art in the field of the invention. Acknowledgement of these references herein will at times be made by indicating their number(s) from the list below next to specific passages herein.
1. Varga, K., Lake, K., Martin, B.R. & Kunos, G. Novel antagonist implicates the CB1 cannabinoid receptor in the hypotensive action of anandamide. European Journal of Pharmacology 278:279-283 (1995).
2. Pacher, P., Batkai, S. & Kunos, G. Cardiovascular pharmacology of cannabinoids. Handb Exp Pharmacol, 599-625 (2005).
3. Ugdyzhekova, D.S. et al. Activation of cannabinoid receptors decreases the area of ischemic myocardial necrosis. Bull Exp Biol Med 133:125-6 (2002).
4. Krylatov, A.V. et al. Activation of type II cannabinoid receptors improves myocardial tolerance to arrhythmogenic effects of coronary occlusion and reperfusion. Bull Exp Biol Med 131:523-5 (2001).
5. Ugdyzhekova, D.S. et al. Endogenous cannabinoid anandamide increases heart resistance to arrhythmogenic effects of epinephrine: role of CB(1) and CB(2) receptors. Bull Exp Biol Med 131:251-3 (2001).
6. Bouchard, J.-F., Lepicier, P. & Lamontagne, D. Contribution of endocannabinoids in the endothelial protection afforded by ischemic preconditioning in the isolated rat heart. Life Sciences 72:1859-1870 (2003).
7. Joyeux, M. et al. Endocannabinoids are implicated in the infarct size-reducing effect conferred by heat stress preconditioning in isolated rat hearts. Cardiovascular Research
8. Wagner, J.A., Jarai, Z., Batkai, S. & Kunos, G. Hemodynamic effects of cannabinoids: coronary and cerebral vasodilation mediated by cannabinoid CB1 receptors. European Journal of Pharmacology 423:203-210 (2001).
9. Wagner, J.A. et al. CB1 cannabinoid receptor antagonism promotes remodeling and cannabinoid treatment prevents endothelial dysfunction and hypotension in rats with myocardial infarction. 138:1251-1258 (2003).
10. Mechoulam, R. & Hanus, L. Cannabidiol: an overview of some chemical and pharmacological aspects. Part I: chemical aspects. Chem Phys Lipids 121:35-43 (2002).
11. Cunha, J.M. et al. Chronic administration of cannabidiol to healthy volunteers and epileptic patients. Pharmacology 21, 175-85 (1980).
12. Brady, C.M. et al. An open-label pilot study of cannabis-based extracts for bladder dysfunction in advanced multiple sclerosis. Mult Scler 10:425-33 (2004).
13. Rog, D.J., Nurmikko, T.J., Friede, T. & Young, C.A. Randomized, controlled trial of cannabis-based medicine in central pain in multiple sclerosis Neurology 65:812-819 (2005).
14. Zajicek, J. et al. Cannabinoids for treatment of spasticity and other symptoms related to multiple sclerosis (CAMS study): multicentre randomised placebo-controlled trial. The Lancet 362:1517-1526 (2003);
15. Bisogno, T. et al. Molecular targets for cannabidiol and its synthetic analogues: effect on vanilloid VR1 receptors and on the cellular uptake and enzymatic hydrolysis of anandamide. Br J Pharmacol 134:845-52 (2001).
16. Pertwee, RG., Thomas, A., Stevenson, L.A., Maor, Y. & Mechoulam, R. Evidence that (-)-7-hydroxy-4'-dimethylheptyl-cannabidiol activates a non-CB(1), non-CB(2), non-TRPV I target in the mouse vas deferens. Neuropharmacology 48:1139-46 (2005).
17. Carrier, E.J., Auchampach, J.A. & Hillard, C.J. Inhibition of an equilibrative nucleoside transporter by cannabidiol: A mechanism of cannabinoid immunosuppression PNAS 103, 7895-7900 (2006).
18. Hampson, A. J., Grimaldi, M., Axelrod, J., and D. Wink. Cannabidiol and (-)Δ9-tetrahydrocannabinol are neuroprotective antioxidants. Proc Natl Acad Sci USA 95(14):8268-8273 (1998);
19. [Watzl, B., Scuderi, P. and Watson, R R. Influence of marijuana components (THC and CBD) on human mononuclear cell cytokine secretion in vitro. Adv Exp Med Biol 288:63-70 (1991);
20. Steffens, S., Veillard, N. R., Arnaud, C., Pelli, G., Burger, F., Stauf C., Zimmer, A., Frossard, J.-L., and Mach, F. Low dose oral cannabinoid therapy reduces progression of atherosclerosis in mice. Nature 434:782-786 (2005);
21. Mechoulam R, Shani A, Edery H, Grunfeld Y. Chemical basis of hashish activity. Science 169:611-612 (1970);
22. Marijuana/ cannabinoids: neurobiology and neurophysiology, ed. L. Murphy and A. Bartke, CRC Press, Boca Raton, pp. 1-33 (1992);
23. Giugliano, G.R., Giugliano, R.P., Gibson, C.M. & Kuntz, R.E. Meta-analysis of corticosteroid treatment in acute myocardial infarction. The American Journal of Cardiology 91:1055-1059 (2003).
24. Dr. Xu Z, Mueller RA, Park SS, Boysen PG, Cohen MV, Downey JM. Cardioprotection with adenosine A2 receptor activation at reperfusion J Cardiovasc Pharmacol. 46(6):794-802 (2005).
25. Headrick JP, Peart J. A3 adenosine receptor-mediated protection of the ischemic heart Vascul Pharmacol. 42(5-6):271-9 (2005).
26. Hayakawa et al, Cannabidiol prevents infraction via the non-CB1 cannabinoid receptor mechanism, Neuroreport, 15(15):2381-5 (2004).

### BACKGROUND OF THE INVENTION

Cannabinoids are natural and synthetic compounds structurally or pharmacologically related to the constituents of the plant *Cannabis sativa* or to the endogenous agonists (endocannabinoids) of the cannabinoid receptors CB1 or CB2 [Pertwee, R.G. Pharmacological actions of cannabinoids. Handb Exp Pharmacol, 1-51 (2005); Mechoulam, R. Cannabinoids as Therapeutics, ed. Mechoulam, R. (Birkhauser Verlag, Basel, 2005); Onaivi, E.S., Sugiura, T., Di Marzo, V. Endocannabinoids - The Brain and Body's Marijuana and Beyond. (Taylor and Francis, London, 2006. Most of the research on this group of compounds has been carried out either on the psychoactive plant component Δ⁹-tetrahydrocannabinol (THC) [Gaoni, Y., Mechoulam, R. Isolation, structure and partial synthesis of an active constituent of hashish. J. Amer. Chem. Soc. 86, 1646-1647 (1964)] or on the endocannabinoids anandamide [Devane, W.A. et al. Isolation and structure of a brain constituent that binds to the cannabinoid receptor. Science 258, 1946-9 (1992)] and 2-arachidonoylglycerol (2-AG) [Mechoulam, R. et al. Identification of an endogenous 2-monoglyceride, present in canine gut, that binds to cannabinoid receptors. Biochem Pharmacol 50, 83-90 (1995); Sugiura, T. et al. 2-Arachidonoylglycerol: a possible endogenous cannabinoid receptor ligand in brain. Biochem Biophys Res Commun 215, 89-97 (1995)].

In a rat model it was shown that anandamide has a physiological triphasic effect on the cardiovascular system consisting of initial reduction in heart rate and blood pressure, followed by a brief pressor response, and a third prominent phase of reduction in both blood pressure and heart rate¹. Indeed, cannabinoid receptor mRNA and endocannabinoids have been detected in rat myocardium². It has been shown that endocannabinoids have a protective effect against myocardial ischemia and can help preserve coronary endothelial function during ischemia. These effects are receptor-mediated and can be inhibited by specific CB1 and CB2 receptor blockers³⁻⁵. In one study, HU-210 (a potent CB1 and CB2 agonist) was shown to substantially reduce the myocardial necrotic zone after LAD ligation³, whereas in another trial HU-210 was shown to significantly reduce the ischemic arrhythmic effect in treated animals. These effects were mediated by the CB2 receptor^{4,5}. Other studies have shown that the cardioprotective effect of heat and ischemic preconditioning is mediated, at least in part, by CB2 receptors, and can be abolished by CB2 antagonists^{6,7}. HU-210 was also shown to increase coronary blood flow and to be associated with reduced remodeling in infarcted rat hearts^{8,9}. These results indicate a possible role for the endocannabinoid system in stress-induced preconditioning.

Cannabidiol (CBD) is a major cannabinoid constituent of *Cannabis* species, such as the hemp plant (*Cannabis sativa*). Unlike THC, cannabidiol binds very weakly to CB 1 and CB2 receptors¹⁰. CBD does not induce psychoactive or cognitive effects and is well tolerated without side effects in humans^{10,11}, thus making it a putative therapeutic target.

CBD has been shown to have anti-inflammatory properties [Croxford, J.L. & Yamamura, T. Cannabinoids and the immune system: potential for the treatment of inflammatory diseases? J Neuroimmunol 166:3-18 (2005)], to suppress pathologic manifestations of autoimmune diseases in animal models of arthritis [Malfait, A.M. et al. From the Cover: The nonpsychoactive cannabis constituent cannabidiol is an oral anti-arthritic therapeutic in murine collagen-induced arthritis PNAS 97;9561-9566 (2000)] and β-cell destruction due to insulitis.^{[} Weiss, L. et al. Cannabidiol lowers incidence of diabetes in non-obese diabetic mice. Autoimmunity 39:143-51 (2006)] and to suppress macrophage nitric oxide (NO) production and T-cell proliferation²². CBD (together with THC) has been successfully tested in a few preliminary human trials related to autoimmune diseases such as rheumatoid arthritis [Blake, D.R., Robson, P., Ho, M., Jubb, R.W. & McCabe, C.S. Preliminary assessment of the efficacy, tolerability and safety of a cannabis-based medicine (Sativex) in the treatment of pain caused by rheumatoid arthritis Rheumatology 45:50-52 (2006)] and multiple sclerosis.

CBD has also been shown to be superior to another cannabinoid, Δ9-tetrahydrocannabinol (THC), in inhibiting pro-inflammatory IL-1, TNFα and IFNγ release by peripheral blood mononuclear cells¹⁹. THC, the major constituent of marijuana, which binds to both the CB1 and CB2 cannabinoid receptors and is thus responsible for its psychotropic (drug) effects, was found to induce an anti-atherosclerotic effect *in vivo* at a low dose²⁰.

The mechanisms of action of CBD are still not fully understood. It is known that CBD has a mild effect on VR1 receptors^{15,16}. Recently it was proposed that the anti-inflammatory effects of CBD might be mediated by enhancement of adenosine signaling through inhibition of its uptake, the effect apparently involving the A_{2A}-adenosine receptor (A2AR)¹⁷]. Further, The A₃ adenosine receptor seems to have multiple beneficial effects on ischemic-reperfusion injury, including modulation of necrotic and apoptotic cell death and enhancement of contractile function²⁵. The A_{2A} adenosine receptor may also play a role in protection from ischemia²⁴.

Unlike other cannabinoids, cannabidiol does not bind CB1 or CB2, or its binding to the receptors is negligible in terms of inducing a biochemical effect. Thus, cannabidiol does not cause the central or peripheral nervous system effects mediated by the CB1 or CB2 receptors. CBD has no psychotropic (cannabimimetic) activity and its molecular structure and properties are substantially different from those of other cannabinoids^{21,22}.

The use of cannabidiol for treating inflammatory diseases such as rheumatoid arthritis, multiple sclerosis and Crohn's disease, and medicinal preparations comprising CBD for such uses, has also been described [US patent No. 6,410,588]. In addition, pharmaceutical compositions comprising cannabidiol derivatives which have analgesic, anti-anxiety, anti-convulsive, neuroprotective, antipsychotic and anti-cancer activities have been described [PCT patent application publication no. WO 01/95899; and Mechoulam, R., Parker, L. A., and Gallily, R Cannabidiol: an overview of some pharmacological aspects. J Clin Pharmacol 42:11S-19S (2002)].

Atherosclerosis is a chronic inflammatory condition often leading to acute disease symptoms with plaque rupture and thrombosis [Libby, P. Inflammation in atherosclerosis. Nature 420:868-874 (2002)]. Atherosclerosis is the leading cause of heart disease and stroke among Western populations. Current treatment regimens are based on drugs for lowering blood pressure and plasma cholesterol levels. HMG-CoA reductase inhibitors (statins) reduce cardiovascular events by lowering cholesterol and perhaps inducing anti-inflammatory and immunomodulatory effects [Mach, F. Statins as immunomodulatory agents. Circulation 109 suppl., II15-II17 (2004)].

Lipid profile is a term used to describe the pattern of lipids in the blood. A lipid profile usually includes the total cholesterol, high density lipoprotein (HDL) cholesterol, triglycerides, and the calculated low density lipoprotein (LDL) cholesterol, and at times the ratio of HDL to LDL levels. The lipid profile has been tightly correlated with various cardiovascular conditions in particular coronary atherosclerosis.

Acute myocardial infarction (AMI or MI), commonly known as a heart attack, occurs when the blood supply to a part of the heart is interrupted, causing death and scarring of the local heart tissue. Since the area affected may be large or small, the severity of heart attacks vary, but they are often a life-threatening medical emergency which demand both immediate attention and activation of the emergency medical services.

The most important treatment in myocardial infarction is restoring the blood flow to the heart, by thrombolysis (enzymatically dissolving the clot in the artery) and/or angioplasty (using a balloon to push the artery open).

In the past corticosteroids were studied as a potential therapy for myocardial infarction due to their anti-inflammatory effect²³. However, this therapy has ultimately turned out to be harmful as corticosteroids delay and interfere with myocardial scar formation.

### SUMMARY OF THE INVENTION

In accordance with its broadest aspect, the present invention provides the use of a cannabidiol (CBD) compound for the preparation of a pharmaceutical composition for treatment of at least one fundamental parameter affecting a vascular system selected from (a) the cardiovascular system; (b) the peripheral vascular system; or (c) a combination of (a) and (b), the fundamental parameter selected from at least one of (i) dimension of heart scars, (ii) blood/plama lipid levels (iii) atheroselerosis.

By a preferred embodiment the present invention excludes the use of CBD for the preparation of a composition which affects the vascular system of the central nervous system (herein the CNS vascular system). Thus, exclusion is also made to the use of CBD for the preparation of compositions to prevent neuronal death or damage due to CNS ischemia, which is known to result from low oxygen supply to neurons.

A most preferred embodiment in accordance with this aspect of the invention is the use of CBD in the preparation of a composition for the treatment of heart scars.

In accordance with another aspect, the invention provides a biocompatible device comprising a CBD compound, the device being configured for deployment into a subject's vascular system selected from the cardiovascular system, the peripheral vascular system or both, such that upon deployment of the device in the subject's vascular system, the CBD compound is released from the device in an amount effective to treat or prevent at least one fundamental parameter affecting the vascular system.

### BRIEF DESCRIPTION OF THE DRAWINGS

In order to understand the invention and to see how it may be carried out in practice, a preferred embodiment will now be described, by way of non-limiting example only, with reference to the accompanying drawings, in which:
**Figure 1** is a bar graph showing LDL cholesterol levels in ApoE^{-/-} mice after intraperitoneal injections with a control (Ctrl- Cremophor:ethanol:saline 1:1:18), or with CBD;
**Figure 2** is a bar graph showing of heart scar size measured as percent of area at risk measured by planimetry using TTC staining. P< 0.01 for comparing scar size of CBD-treated and control animals using the unpaired, two-tailed T-test;
**Figures 3A-3B** are photograph images of slices of the hearts of rats injected IP prior to and post-heart surgery, with a control solution (Cremophor:ethanol:saline 1:1:18) (Fig. 3A) or with CBD (Fig. 3B), where white areas represent the infarcted area, and gray areas represent areas at risk;

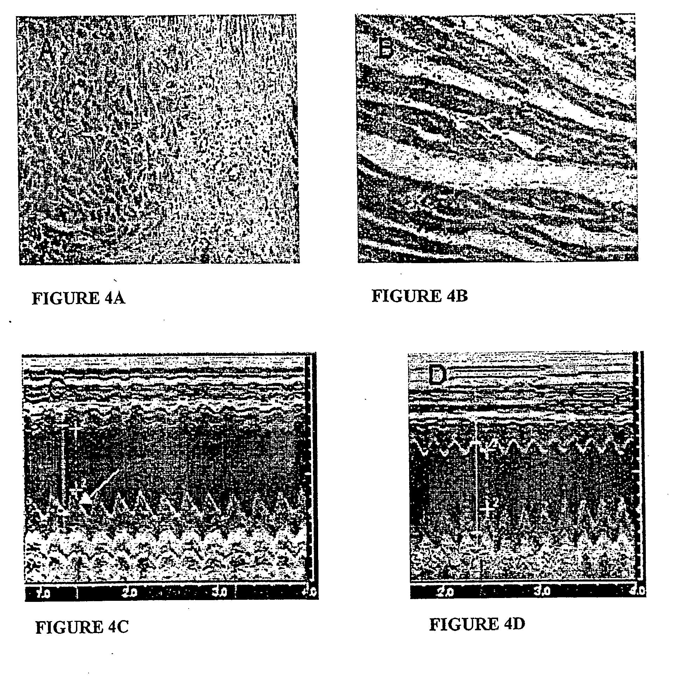

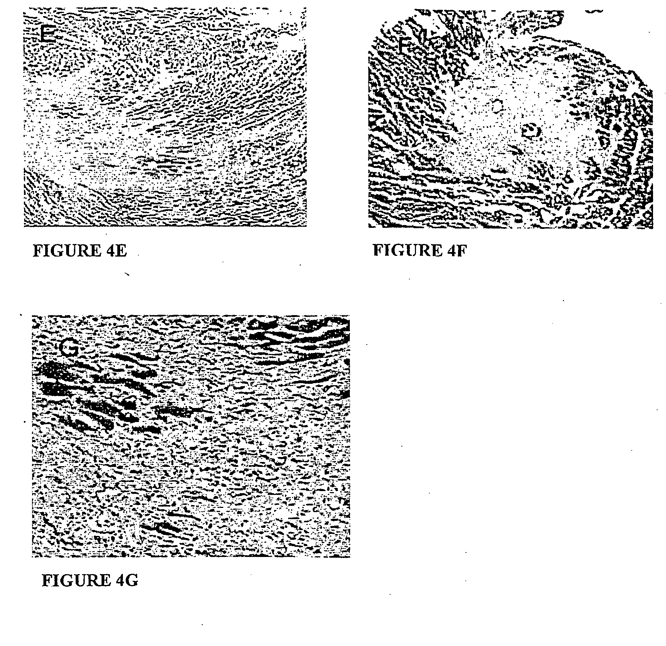

**Figures 4A-4G** are M mode echocardiographs and histological analysis of CBD-treated and control rats, showing marked inflammation in a typical control animal (Fig. 4A); an almost total lack of inflammation in an animal treated with CBD (Fig. 4B); M mode echocardiography of a sham-treated rat with reduced shortening fraction and reduced motion of the anterior wall (arrow) (Fig. 4C); a CBD-treated animal with preserved LV function and preserved motion of the anterior wall (Fig. 4D); early collagen deposition in the infarcted area in a control animal (Masson trichrome x 40) (Fig. 4E); early collagen deposition in the infarcted area in a CBD-treated animal, showing similar staining in the two groups (Masson-trichrome x 40) (Fig. 4F), and a higher magnification view from a control animal showing replacement of necrotic tissue by early fibrovascular granulation tissue (Fig. 4G).

### DETAILED DESCRIPTION OF THE INVENTION

The present invention is based on *in vivo* experiments showing that a cannabidiol (CBD) compound was effective in reducing total area covered by atherosclerotic plaques (composed of the number and/or size of atherosclerotic plaques) in aortas and in lowering triglycerides, LDL and cholesterol levels.

The present invention is further based on the finding that *in vivo* administration of CBD reduced the scar size in rat hearts after a surgically induced infarct.

The effect of CBS is surprising, particularly in view of the fact that CBD binds very weakly to CB1 or CB2 receptors¹⁰, the latter shown to be related to endocannabinoids' protective effect against myocardial ischemia and preservation of coronary endothelial function during ischemia (the effects of the endocannabionoids, shown to be receptor-mediated, can be inhibited by specific CB 1 and CB2 receptor blockers³⁻⁵).

Thus, the present invention provides the use of a CBD compound for the preparation of a pharmaceutical composition for the treatment of at least one fundamental parameter affecting a vascular system selected from (a) the cardiovascular system; (b) the peripheral vascular system or (c) a combination of (a) and (b), the fundamental parameter being selected from at least one of (i) dimension of heart scars, (ii) blood/plasma lipid levels; (jii) atherosclerosis.

When referring to the treatment of *"the cardiovascular system"* and/or *"the peripheral vascular system"* it is meant any effect that changes a structural, histological, physiological function, and/or systemic function of the heart and/or the veins or arteries within the vascular system or of the blood itself. Thus, it is to be understood that the vascular system in the context of the present invention refers both to the cardiovascular (coronary) system as well as the peripheral vascular system. The term refers thus to the heart, the blood and the blood vessels.

In connection with the blood, the term ***"treatment"*** concerns improvement in blood profile and in particular in blood lipid profile improvement includes on the one hand reduction or prevention of elevation of a lipid level, the lipid selected from triglycerides, low density lipoprotein (LDL) and/or cholesterol, as well as the increase or prevention of decrease in high density lipoprotein levels or in the HDL/LDL ratio. In connection with blood vessels (both coronary and peripheral), the term **"*treatment*"** concerns the widening of blood vessels so as to restore or improve flow of blood through them, as well as improving the functions and/or integrity of the endothelia forming the wall of the blood vessels and most preferably in performing the above two effects by decreasing plaque load in the blood vessels, or by improving blood/plasma lipid profile.

The term **"*fundamental parameter affecting the vascular system*"** refers to a physiological, histological or structural parameter that may be medicinally or surgically manipulated to improve cardiovascular and/or peripheral vascular performance (i.e, the functions of the coronary and peripheral vascular systems, the function of the heart or the blood/plasma profile). While many parameters are known to be linked to cardiovascular and as well as to peripheral vascular diseases, and have an indirect effect in improving it to some extent, today there are defined several central parameters which are clinically accepted as the key parameters to be manipulated and having a direct effect on these functions. The term "*fundamental*" refers to these central parameters.

These parameters include, in accordance with the invention: cardiac function in general; dimension of the heart scar (e.g. size or thickness of heart scar) and cardiac function in general following damaging events such as myocardial infarct or as a result of a coronary heart disease (CHD); blood/ plasma lipid profile modification; and atherosclerosis plaque load The CBD compound in accordance with the invention is used in order to treat (e.g. improve) or prevent one or more of these parameters.

In accordance with the invention, the individual fundamental parameters are to be understood as having the following meanings:

**"*Myocardial scar size*" *-*** the heart scar dimensions including the size, thickness, and/or the combined area of several scars, formed due to trauma to the heart, for example due to deprivation of blood flow to the heart (such as that that occurs during myocardial ischemia) as well as due to other causes such as vasospasm, embolization, dissection, Kawasaki disease, catheterization complications and/or inflammation such as viral myocarditis, SLE and sarcoidosis

**"*Blood*/*plasma lipid profile*" *-*** blood or plasma (whichever is examined) levels of high density lipoprotein (HDL), low density lipoprotein (LDL), triglycerides and/or total cholesterol levels, as well as to the HDL/LDL ratio. Preferably this term refers to levels of LDL, triglycerides and total cholesterol. It is noted that elevated blood lipid levels (e.g. high triglyceride levels as well as high blood cholesterol (LDL) levels) are risk factors for developing atherosclerosis.

**"*Atherosclerosis plaque load*" *-*** total blood vessel area covered by plaques, which is a combination of the number of plaques and the size of each plaque. This term also refers to the rate of formation of new plaques (the number and size of the new plaques resulting in total area) and rate of build-up of plaques increasing the size and/or thickness of existing plaques.

**"*Cardiac function*" *-*** the global systolic and diastolic function of the of heart measured in terms of cardiac output, ejection fraction (the ratio of blood ejected to the total blood contained in the ventricle at end diastole), stroke volume, rate of fiber shortening, stroke work, diastolic function as measured by left ventricular inflow velocities, pulmonary vein flow rates, tissue Doppler velocities and left ventricular compliance.

As stated above, the invention concerns the use of a CBD compound for treating at least one fundamental parameter affecting the vascular system of a subject. Treatment, in the context of the invention, comprises any improvement in such fundamental parameters, i.e. any change of one or more of the said parameter(s) such that the determined parameter becomes closer to the corresponding parameter in a healthy subject or closer to the parameter determined or believed by the medical community to be desirable. Treatment also includes prophylactic treatment (i.e. preventative treatment).

In connection with treating the *myocardial scars,* the term "*improvement*" denotes reduction in the size of the scars, as well as a improvement in other dimensions of the infarction such as thickness of affected myocardial tissue and a reduction in the number and volume of scars, as well as to improvement in cardiac function, in general. In this context, the term may also refer to treatment of an already existing scar, such as speed-up of healing, change in the severity of the scar (e.g. change in length and width, dimension, thickness of viable myocardial tissue and other parameters defining the scar) etc.

In connection with treatment of *blood*/*plasma lipid profiles,* the term "*improvement*" comprises any change in the parameter to that which is closer to pre-determined normal levels (as known to those in the art), e.g. lowering LDL, total cholesterol and/or triglyceride levels, and at times increasing HDL levels or increasing the HDL/LDL ratio. The improvement may also concern prevention of the deterioration of the blood lipid profile of a subject who may already be diseased, or of a healthy subject.

In connection with treatment of *atherosclerosis plaque load,* the term "*improvement*" comprises any decrease in the number, size or thickness of plaques, or decrease in the rate of formation of new plaques (number and/or size and/or dimension) or decrease in the rate of build-up of already existing plaques (increase in their size and/or thickness). This term may refer to prevention of formation of new plaques.

In connection with treatment of *cardiac function,* the term "*improvement*" comprises any change in one or more of the performance parameters selected from cardiac output, ejection fraction, fiber shortening rate/fraction, stroke word/volume etc., so that the performance of any one of these parameters becomes closer to the corresponding parameter in a healthy subject or closer to a parameter determined or believed by the medical community to be desirable.

Thus, with respect to the above, the CBD compound may be used for the preparation of a pharmaceutical composition for any one or more of the following indications:
- for the treatment of heart scars or the prevention of formation of heart scars;
- for reducing or preventing elevation of blood/plasma lipid levels; the blood/plasma lipid levels comprising, without being limited thereto, triglyceride levels, low density lipoprotein (LDL) levels, LDL/HDL ratio and/or total cholesterol level;
- for reducing atherosclerosis plaque load or preventing the build-up of atherosclerosis plaques on the internal walls of blood vessels;
- for improving cardiac function;
- for the treatment or prevention of atherosclerosis.

A preferred embodiment of the invention comprises the use of a CBD compound for the preparation of a pharmaceutical composition for the treatment of heart scars or for the prevention of heart scar formation. Preferably, the invention is applicable to treatment of heart scars formed following myocardial ischemia and more preferably due to myocardial infarct.

The pharmaceutical preparation may be used to prevent or decrease the formation of the scar, or to improve scar parameters of an already existing scar, all being determined in accordance with medical practice²⁶.

The present invention discloses, for the first time, the protective effect of CBD on the vascular system, via its direct effect on blood lipid profile (lipids, cholesterol etc.) on the blood vessels and on the formation of infarcts which has nothing to do with neuronal functionality. Thus, the affect of CBD on neurons and thereby on vascular system of the CNS (which may be associated with CNS ischemia) does not form part of the invention.

In accordance with a preferred embodiment, the invention concerns the protective effect of CBD on the cardiovascular system, most preferably, on the formation of myocardial infarcts.

It should be appreciated that in the context of the present invention the terms **"*Cannabidiol compound*"***, **"cannabidiol"*** or **"*CBD compound*"** (which may be used interchangeably unless the context clearly dictates otherwise) refer to any natural, semisynthetic or synthetic cannabinoid compound.

According to one embodiment, the CBD compound comprises the following general Formula (I): 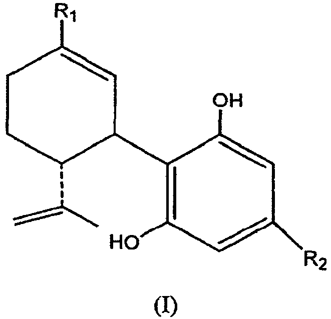 wherein
**R₁** is an alkyl; and
**R₂** is selected from a straight or branched alkyl having 5 to 12 carbon atoms; an-OR₃ group, wherein **R₃** is a straight or branched alkyl having 5 to 9 carbon atoms or a straight or branched alkyl substituted at the terminal carbon atom by a phenyl group; or a-(CH₂)ₙ-O-alkyl group, wherein n is an integer from 1 to 7 and the alkyl group has 1 to 5 carbons.

In one preferred embodiment, R₁ is CH₃ and R₂ is a straight alkyl having 5 carbon atoms (i.e. -C₅H₁₁).

In another preferred embodiment, the CBD compound is cannabidiol. Cannabidiol has the following formula (II):

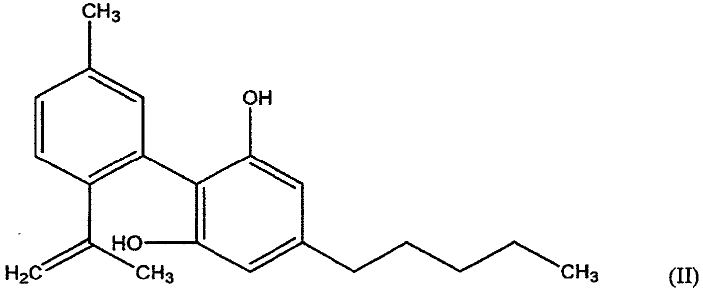

The cannabidiol of formula (II) may be a natural cannabidiol obtainable by extraction from a plant member of the genus *Cannabis* or any preparations of *Cannabis* (e.g., processed plant material). According to one embodiment, the natural cannabidiol may be extracted from *Cannabis sativa* or one of its preparations, e.g., marijuana, hashish, etc. According to one embodiment, the natural cannabidiol can be extracted from *Cannabis* using methods such as described, for example, in U.S. Pat. No. 6,403,123, and in Gaoni and Mechoulam [J Am Chem Soc 93:217-224 (1971)], both incorporated herein by reference.

The cannabidiol may also be a synthetic cannabidiol or a derivative thereof which can be generated using methods such as those described, for example and without being limited thereto, in WO01/95899 (corresponding to US 2003/166727, and incorporated herein by reference).

In accordance with one aspect of the invention, the CBD compound is used in combination with an invasive cardio- or coronary procedure.

As will be further detailed below, CBD may be used for the preparation of compositions for systemic as well as for local therapy.

In accordance with this aspect, the CBD compound may be administered in conjunction with (i.e. before, during and/or after) an interventional procedure, or as part of the procedure, as will be explained below, preferably for local treatment at the site of the vascular disease or injury (e.g. the site which requires an improvement of at least one of the aforementioned fundamental parameters affecting the vascular system).

When the CBD compound or a composition comprising it is administered as part of the interventional procedure (which may be an invasive cardiac, coronary or peripheral procedure, as known to those versed in the art), it is preferably meant, in the context of the present invention that it is constructed as part of an implantable device which may be inserted into a subject's body during the procedure.

Thus, the invention also provides the use of a CBD compound as part of an implantable device, for example present in a coat on said device and capable of controlled or prolonged release therefrom. In accordance with this embodiment of the invention, the implantable device is preferably a biocompatible device comprising (for example coated by a coat comprising) the CBD compound and configured for deployment into the subject's vascular system (the vascular system being selected from the cardiovascular system, the peripheral vascular system or both), such that upon deployment of the device in said subject's vascular system, the CBD compound, or a pharmaceutical composition comprising the same, is released from the device in an amount effective to treat or prevent at least one fundamental parameter affecting the vascular system as described above. The device may act as a carrier per se, e.g. being inserted into the target area within the vascular system for purposes of delivery of the CBD compound only; however, it may have a dual effect, including delivery of the drug on the one hand, and supporting the diseased or injured target site on the other hand

Device implantation is frequently used to treat various vascular diseases, such as stenosis/restenosis, atherosclerosis, acute myocardial infarction, coronary heart disease (CHD), etc. Each particular disease is characterized by different types of occlusions and pathologies, often involving different cell types and extracellular components. For example, stenosis is a narrowing or constricting of arterial lumen usually due to atherosclerosis/coronary heart disease (CHD). Restenosis is a recurrence of stenosis after a percutaneous intervention such as angioplasty and/or stent implantation.

An implantable (intravascular) device in accordance with the invention is preferably to be understood as any prosthesis which may be placed within a body passageway such as a vein or artery. Typically, the device is inserted into a vessel and placed at a site of therapeutic interest (e.g. a site of vascular occlusion or injury).

The device in accordance with the invention may thus have any shape suitable for insertion into the vascular system without damaging the walls of the vessel. In accordance with some embodiments, the device comprises, without being limited thereto, a radially expandable wire, stent or a balloon; perforated tube; catheter of any size and shape, intravascular needle or an ostial stent or balloon, as known in the art.

The device is to be deployed within the target vessel or other vasculature component by the use of suitable deployment systems known for implantation. A non-limiting list of deployment systems includes catheters including balloon catheters and intravascular needles.

Delivery and deployment of a device may be accomplished by positioning the device about one end of a catheter, inserting the end of the catheter through the skin into a bodily lumen, advancing the catheter in the bodily lumen to a desired treatment location, expanding the device at the treatment location, and removing the catheter from the lumen. Once in place and released from the deployment system, the implantable device may expand in order to contact the vessel's wall, e.g. the vessel or other components of the vascular system, thereby widening the vessel and providing mechanical support for the wall. The device may be self-expanding or may expand by the use of expanding means, such as a balloon catheter that provides pressure from within the device outwards thereby pressing the device against the walls of the passageway.

The device may be coated with the CBD composition (or the compound per se), or with a coat such as a biocompatible polymers comprising the CBD composition/compound in a releasable form , impregnated with the CBD composition/compound, or the composition may be sequestered in holes, grooves, or pores of the device or sequestered within an inner space of the device (e.g. housed within the lumen of a tubular device), or the device may be integrally formed with the CBD compound/composition, all being in a manner resulting in the *in situ* controlled release of that CBD compound from the device, at least at the area of insertion of the device in the vascular system.

The device should preferably be able to satisfy a number of mechanical requirements. First, the device must be capable of withstanding the structural loads, namely radial compressive forces, imposed on the device as it supports the walls of a vessel. Therefore, the device must possess adequate radial strength. Radial strength, which is the ability of the device to resist radial compressive forces, is due to strength and rigidity around a circumferential direction of the device.

Once expanded, the device should preferably adequately maintain its size and shape throughout its service life despite the various forces that may come to bear on it, including the cyclic loading induced by the beating heart. For example, a radially directed force may tend to cause a device to recoil inward.

In addition, the device should preferably possess sufficient flexibility to allow for crimping, expansion, and cyclic loading. Longitudinal flexibility is important to allow the device to be maneuvered through a tortuous vascular path and to enable it to conform to a deployment site that may not be linear or may be subject to flexure. Finally, the device should preferably be biocompatible so as not to trigger any adverse vascular or immunological responses.

The device may be made of a variety of materials as known to those versed in the art of interventional cardiology. For example, and without being limited thereto, the device may be manufactured from a multitude of metals (alloys) including stainless steel, cobalt chromium, magnesium, and nickel-titanium, commonly referred to as nitinol. Nitinol is a self-expanding memory metal. The device may also be manufactured from other materials, for example polymers or self-degradable materials such as lactic acid materials or derivatives thereof, which may be typically combined with the metal alloy. The device may be manufactured from biodegradable polymers. Biodegradable devices may be desirable in some treatment applications, such as when the presence of the device in the vessel may be necessary for a limited period of time, e.g. until the delivery of the CBD compound is complete.

The composition prepared from the (CBD compound may be used for systemic as well as for local therapy and the mode of administering the compound may be modified in accordance with its intended use.

Preferably, the pharmaceutical composition is formulated such that the CBD compound, acting as the active ingredient, is combined with pharmaceutically acceptable carriers and excipients. The CBD compound may be combined with other active ingredients; however, the CBD compound at least would be accountable for the intended therapeutic effect as defined herein.

The pharmaceutical composition to be used according to the invention may be applicable for any one of the indications detailed above, including treatment or prevention of the formation of infarcts (preferably, myocardial infarcts), improvement of lipid blood levels, treating or preventing atherosclerosis, etc. A preferred pharmaceutical composition is a composition comprising as an active ingredient the CBD compound which may be used for the treatment of heart scars, e.g. following myocardial infarct, as well as to treat already existing infarcts.

The pharmaceutical composition may be formulated so as to allow the controlled release of the CBD compound from the pharmaceutical composition, once administered to the subject. The term *"controlled release"* denotes the manipulation of a desired release profile of the CBD compound within the subject's vascular system, including, without being limited thereto, sustained therapeutic release, immediate therapeutic release, slow therapeutic release or a combination of same.

It is noted that when using an implantable device as a carrier for the CBD compound or composition, a sustained local as well as systemic therapeutic release profile may be achieved, for example, by the use of an intravascular stent; an immediate therapeutic release may be achieved by, e.g., the use of a balloon catheter or an intravascular injection system; and a combined release profile may be achieved, for instance, by using a stent having a rapid release, CBD-impregnated coating over a delayed/sustained release CBD-impregnated inner coating.

As used herein, the term *"subject"* denotes a mammalian individual, more preferably a human.

As used herein, the term *"physiologically acceptable carrier",* which may be used interchangeably with the term *"pharmaceutically acceptable carrier",* refers to a carrier or any other pharmaceutically/physiologicaly acceptable excipient mixed with the CBD compound so as to facilitate the delivery of the CBD compound (and other active ingredients, if present in the composition) to the target site and that does not abrogate the biological/therapeutic activity and properties of the CBD compound (and, if applicable, of other active ingredients). While the pharmaceutically acceptable carrier is preferably an inert substance added to the composition, adjuvants (i.e. agents which enhance the bioiogical/therapeutic activity of the active ingredient) are also included under this term.

The carrier can be of any type conventionally used in pharmaceutical practice and may be limited only by chemico-physical considerations, such as solubility and lack of reactivity with the CBD compound, and by the preferred route of administration. The choice of carrier may be determined in part by the active ingredient, e.g. by the chemical and physical characteristics of the CBD compound, the particular method used to administer the compound or composition etc. Accordingly, there is a wide variety of suitable formulations of the pharmaceutical composition of the present invention, for systemic as well as for local therapeutic effect.

As used herein, the term **"*effective amount*"** means an amount of CBD compound effective to improve, as compare to a non-treated control, at least one fundamental parameter affecting the cardiovascular and/or peripheral vascular system, as defined above.

Depending on the severity and responsiveness of the fundamental parameter to be treated, as defined herein, dosing of the pharmaceutical composition can be designed for a single administration or a plurality of administrations, with a course of treatment lasting from perhaps several days to several weeks, or until improvement or even cure is effected. When using the CBD composition/compound in combination with an implantable device, the loading of the CBD compound onto the device (e.g. amount of CBD compound or manner of loading, which may dictate the release profile of the CBD compound) may also be designed depending on the severity and responsiveness of the fundamental parameter.

In accordance with the selected fundamental parameter examined the regime of administration may also vary. In connection with infarct size reduction, administration typically follows AMI and is typically crucial in the first hours and days after the MI. It is noted, however, that administration may also occur prior to MI as part of a primary prevention in atherosclerosis and could also be used to treat chronic atherosclerosis. On the other hand, in connection with treating elevated blood lipid levels or atherosclerotic plaque load, the administration is typically chronic.

Pharmaceutical compositions of the present invention may be manufactured by processes well known in the art, e.g., by means of conventional mixing, dissolving, granulating, dragee-making, levigating, emulsifying, encapsulating, entrapping, or lyophilizing processes. Proper formulation is dependent, inter alia, upon the route of administration chosen and the.

Suitable routes of administration of the pharmaceutical composition comprising the CBD compound for systemic delivery, may include, for example, oral, sublingual, rectal, transmucosal, or parenteral delivery, including intramuscular, subcutaneous, and intramedullary injections, as well as intrathecal, direct intraventricular, intravenous, inrtaperitoneal, intranasal, or intraocular injections.

Alternately, one may administer the pharmaceutical composition in a local rather than systemic manner, for example, via injection of the pharmaceutical composition directly into the diseased or injured area of the vascular system of a subject as well as implantation of a device carrying the CBD compound at the he diseased or injured area of the vascular system.

Pharmaceutical compositions of the present invention may, if desired, be presented in a pack or dispenser device, such as an FDA-approved kit, which may contain one or more unit dosage forms containing the CBD compound as active ingredient, may be accompanied by instructions for administration, and/or may also be accompanied by a notice in a form prescribed by a governmental agency regulating the manufacture, use, or sale of pharmaceuticals, reflective of approval by the agency of the form of the compositions for human or veterinary administration. Pharmaceutical compositions comprising a preparation of the invention formulated in a physiologically acceptable carrier may also be prepared, placed in an appropriate container, and labeled for treatment of one or more of the above indicated fundamental parameters affecting the vascular system.

Throughout the description and embodiments of this specification, the singular forms "***a***", "***an***" and "***the***" include also plural references unless the context clearly dictates otherwise. Thus, for example, a reference to *"**a cannabidiol compound**"* is a reference to one or more such compounds. Throughout the description and embodiments of this specification, the plural forms of words include singular references as well, unless the context clearly dictates otherwise.

Throughout the description and embodiments of this specification, the words "***comprise***" and ***"contain"*** and variations of these words, for example ***"comprising"*** and ***"comprises",*** mean ***"including but not limited to",*** and are not intended to (and do not) exclude other moieties, additives, components, integers, steps, etc.

The invention will now be described by way of non-limiting examples. It is to be understood that the invention is not limited in its application to the details set forth in the following description or exemplified by the examples. The invention is capable of other embodiments or of being practiced or carried out in other various ways. Also, it is to be understood that the phraseology and terminology employed herein is for the purpose of description and should not be regarded as limiting.

### DESCRIPTION OF SOME NON-LIMITING EMBODIMENTS

As illustrated in the Examples described below, a CBD compound for use in accordance with the embodiments of the present invention was effective *in vivo* in reducing the size of atherosclerotic plaques in the aorta.

In a further experiment, CBD was effective in reducing infarction following surgically induced heart ischemia.

### Example 1: ApoE-/- CBD induces reduction of triglyceride levels

### Materials and Methods

ApoE knock-out (ApoE^{-/-}) mice are known to spontaneously develop atherosclerosis, which may be enhanced by a high-fat diet. Twelve- to fourteen-week-old male and female ApoE^{-/-} mice on a C57B1/6 background were fed a high-fat diet (Takland adjusted calories, Western-type diet: 2.1% fat, 15% cholesterol, 19.5% casein) for 12 weeks.

Blood samples were collected in capillary tubes following twelve hours of fasting, and immediately centrifuged for plasma removal. Plasma samples were assayed for total cholesterol using conventional colorimetric assays. Mice were then sacrificed, and their aorta were extracted and examined for the presence and size of atherosclerotic lesions (plaques).

Mice were then treated with a total of 20 intraperitoneal (IP) injections of 5 mg CBD per Kg body weight. Control mice were injected with Cremophor:ethanol:saline 1:1:18. Plasma triglyceride levels were assayed following twelve hours fasting using an enzymatic reagent (Sigma™).

### Results

In the untreated group (control-treated), the mice increased their plasma cholesterol levels and developed atherosclerotic plaques in their aorta. As compared with the control-treated mice, CBD treatment lowered triglyceride levels by 59%

### Example 2: CBD lowers LDL cholesterol levels

### Materials and Methods

Materials and Methods were as described in Example 1. LDL cholesterol levels were calculated using the Friedewald Formula [Friedewald WT, Levy RI, Fredrickson DS. Estimation of the concentration of low-density lipoprotein cholesterol in plasma, without use of the preparative ultracentrifuge. Clin Chem. 18:499-502 (1972)]:

### LDLc=TC-triglycerides levels/5-HDLc

Statistical analysis was by ANOVA test (*ANalysis* Of *VA*riance between groups).

### Results

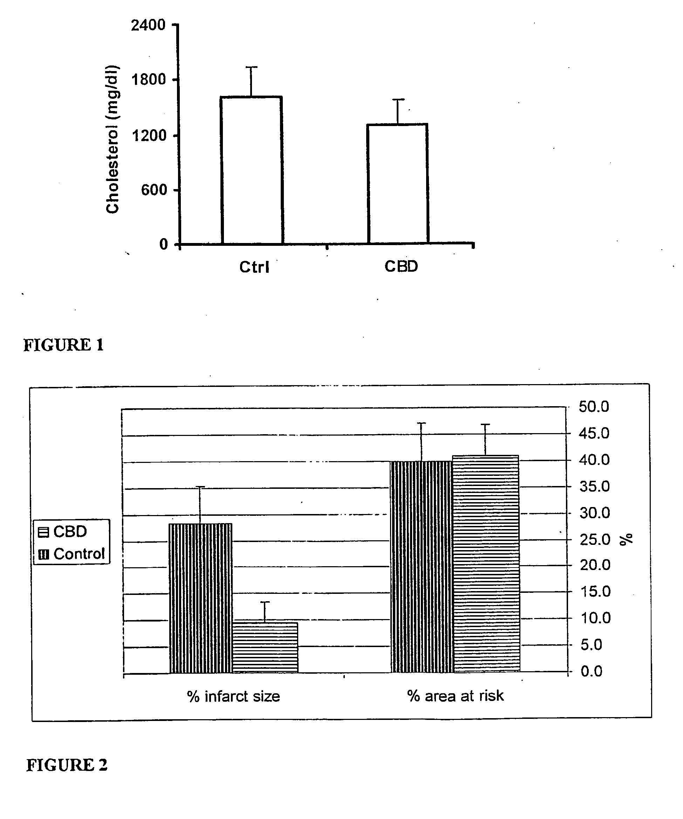

CBD-treated mice had cholesterol levels of 1300 mg/dl (**Fig. 1**). CBD treatment thus compared favorably with the control, in which mice had an average level of LDL cholesterol of 1615 mg/dl. CBD treatment thus lowered LDL levels by 17% (results not shown).

### Example 3: CBD reduces the size of atherosclerotic plaques in the aorta

### Materials and Methods

Materials and Methods were as described in Example 1. Mice were then sacrificed, and their aorta extracted and examined for the size of atherosclerotic plaques, and compared with control (non-CBD)-treated mice.

### Results

A total of 12 CBD-treated and 13 control mice were examined. CBD treatment reduced the plaques from an average of 5.5% of the aorta area in control mice to 3.18% (average). In a second experiment, the plaque area was reduced from 10.6% to 5.09% of the total aorta area. Thus, CBD was shown to reduce the number and size of atherosclerotic plaques *in vivo.*

### Example 4: CBD reduces scar size in rat hearts after surgically induced infarct

### Materials and Methods

### Animals

In the following experiment 14 SD/Hsd male rats weighing 275-350 g were used. The rats were weighed, and CBD or control vehicle (Cremophor:ethanol:saline 1:1:18) was injected IP (5mg/kg) 1 hour prior to surgery and every day for 6 additional days for a total of 7 times.

### CBD preparation

CBD was isolated from hashish as previously described [Gaoni, Y. & Mechoulam, R The isolation and structure of delta-1-tetrahydrocannabinol and other neutral cannabinoids from hashish. J Am Chem Soc 93:217-24 (1971)], dissolved in absolute ethanol, with an equal volume of a detergent (cremophor), and the solution was mixed until homogenous. Saline was added to a final ratio of Cremophor:ethanol:saline 1:1:18. The final concentration used was 5mg/kg body weight

### In vivo LAD ligation surgical procedure

Rats were anesthetized with ketamine (10%): xylazine (2%), 0.85:0.15 IM 0.1 ml/kg. Animals were intubated and ventilated with a small animal respirator (Harvard Sc.). The heart was exposed via left sternotomy and the LAD was reversibly occluded for 30 min.utes using a snare fashioned from an encircling silk ligature and a section of 5 French feeding tube. Coronary occlusion and reperfusion were verified by visual inspection. Following reperfusion the released ligature was left *in situ.* The chest was then closed in layers using uninterrupted sutures. Rats were extubated, received 30-50 cc saline subcutaneously to replace lost body fluids, and were treated with Rymadil (5 mg/kg, i.p.) analgesia for 3 days. The animals were randomizedly selected to receive intaraperitoneal injections of either 5 mg/kg CBD or solvent vehicle as control, 1 hour prior to the procedure and every 24 hours thereafter, for 7 days until sacrifice. The drug and the vehicle solutions were prepared in separate laboratories, ensuring that the staff (e.g. echocardiography and pathology) was blinded to the treatment arm. After 7 days, animals were re-anesthetized and ventilated as described above. The LAD was permanently reoccluded, and the heart harvested for further analysis.

### Echocardiography

Rats were anesthetized with Ketamine (10%):Xylasine (2%), 0.85:0.15 IM 0.1 ml/kg and the chest cavity was shaved. Echo was carried out prior to surgery and 1 week after, prior to sacrifice of the animals.

Echo imaging was performed using a GE Vivid3 platform, equipped with a 13MHz linear epiaortic transducer (General Electric, Haifa, Israel). The probe was positioned in a left parasternal position, and 2D imaging of the heart in the short axis was performed using a high frame rate. This image was used to guide an M-mode cursor down the medial axis of the left ventricle. Measurements were performed in triplicates using the leading edge convention for myocardial borders.

The following parameters were measured: Left ventricular end-diastolic diameter (LVEDD) Left ventricular end-systolic diameter (LVESD); anterior wall thickness in diastole and systole (AWTd, AWTs); and Heart rate (HR).

Shortening fraction (SF) was calculated using the above variables.

### Scar size measurements and histopathology

Measurements were performed as previously described [Nachlas, M.M. & Shnitka, T.K. Macroscopic identification of early myocardial infarcts by alterations in dehydrogenase activity. Am J Pathol 42, 379-405 (1963); Nachlas, M.M., Friedman, M.M. & Cohen, S.P. A Method for the Quantitation of Myocardial Infarcts and the Relation of Serum Enzyme Levels to Infarct Size. Surgery 55, 700-8 (1964); Ytrehus, K. et al. Rat and rabbit heart infarction: effects of anesthesia, perfusate, risk zone, and method of infarct sizing. Am J Physiol 267:H2383-90 (1994)]. Briefly, rats were sacrificed one week after surgery. Animals were again weighed and anesthetized (Ketamine (10%): Xylazine (2%) 0.85:0.15 IM 0.1 ml/kg) and their chest cavity was opened. The LAD was again ligated with the help of the loop that had been left inside. Evans blue (0.5-1 ml of 1% Evans blue solution in saline) was slowly infused retrogradely through the aorta with a catheter (3 ml) into the coronary arteries until the heart turned blue. The heart was then removed, frozen at -20°C for 1 hour in aluminum foil and subsequently cut into 2 mm-thick transverse sections from the occlusion area (LAD) to the apex (usually between 4 to 6 slices). The slices were left to defrost and then washed in PBS to remove any remaining color. The slices were then stained by incubation at 37°C for 15 minutes in 1% W/V triphenyl-tetrazolium chloride (TTC) in phosphate buffer (pH 7.4 (0.5 g in 50 ml PBS)), and fixed in 10% V/V formaldehyde solution.

The slices were then photographed and the area of left ventricle at risk and the area of infarcted tissue in the risk zone were determined by planimetry using Adobe^{©} Photoshop software. Subsequently, tissue slices were fixed in ammonium hydrochloride 5%, paraffin embedded, cut into 5 µm sections and stained with hematoxylin and eosin and Masson trichrome. The extent of the inflammatory response was graded on a 0 - 3 scale by an experienced pathologist who was blinded to the treatment groups.

### Results

Fourteen animals were treated with CBD or vehicle. Their weight, left ventricular end diastolic diameter (LVEDD), left ventricular end systolic dimension (LVESD), anterior wall thickness in diastole (AWTd), and shortening fraction (SF) are presented in **Table 1**.

**Table 1: Baseline and 7 day parameters of CBD and control animals**

| | CBD | | Control | | P value | | | |
|---|---|---|---|---|---|---|---|---|
| | Day 0 | Day 7 | Day 0 | Day 7 | Day 0 vs. 7 (CBD group) | Day 0 vs. 7 (Control group) | Day 0 (between the groups) | day 7 (between the groups) |
| Weight (gr) | 326.79±3.36 | 314.57±8.67 | 326.07±4.32 | 319.71±8.14 | <0.01 | =0.015 | =0.63 | =0.11 |
| LVEDD (mm) | 0.70±0.06 | 0.72±0.05 | 0.67±0.10 | 0.72±0.05 | =0.14 | =0.07 | =0.32 | =0.91 |
| LVESD (mm) | 0.36±0.08 | 0.43±0.07 | 0.37±0.07 | 0.49±0.06 | <0.01 | <0.01 | =0.76 | p=0.041 |
| SF (%) | 48.27±8.6 | 39.7±8.22 | 43.95±5.85 | 32.11±9.12 | =0.02 | <0.01 | =0.26 | P=0.03 |

All animals showed significant weight loss 7 days after the procedure (326.79±3.36gr at baseline to 314.57±8.67 gr and 326.07±4.32 gr to 319.71+8.14 gr, CBD and controls, respectively P<0.05). A significant reduction in SF was observed in all animals after LAD ligation, as expected (from 48.27±8.6% at baseline to 39.7±8.22 % and from 43.95±5.85 % to 32.11±9.12 %, CBD and controls, respectively P<0.05). The left ventricular area at risk, and infarct size as a percent of area at risk, are presented in **Figure 2**.

The area at risk was nearly identical in CBD and control animals. However, the mean infarct size was significantly and remarkably reduced in CBD-treated animals (9.6±3.9% vs. 28.2±7.0% in the CBD and control arm respectively P <0.001). Thus, a relative reduction of 66% in the infarcted zone was observed in the CBD-treated animals.

Similar results are presented in **Table 2** showing the effect of CBD on lowering the infarct size in rat hearts after surgically induced infarct.

**Table 2: Infarct size following CBD treatment**

| Control group* | % infarct size | % area at risk |
|---|---|---|
| | 39.4 | 35.6 |
| | 42.1 | 37.3 |
| | 27.2 | 37.8 |
| | 32.3 | 33.4 |
| | 29.6 | 39.7 |
| | 28.3 | 37.9 |
| | 26.9 | 34.1 |
| | 23.9 | 57.8 |
| | 29.9 | 49.9 |
| | 29.9 | 44.3 |

| CBD-treated group* | | |
|---|---|---|
| | 16.3 | 39.7 |
| | 15.3 | 31.8 |
| | 11.5 | 38.3 |
| | 14.6 | 38.7 |
| | 13.9 | 42 |
| | 6.5 | 41.3 |
| | 7.5 | 33.4 |
| | 7.6 | 41.2 |
| | 7.6 | 46.8 |
| | 8.1 | 40.8 |
| | 7.8 | 55.6 |

| | | |
|---|---|---|
| *each line represents an individual animal | | |

The results presented in **Table 2** and in **Figure 2** show that treatment of rats with CBD before surgery and for 6 days after surgery significantly reduces the size of infarct

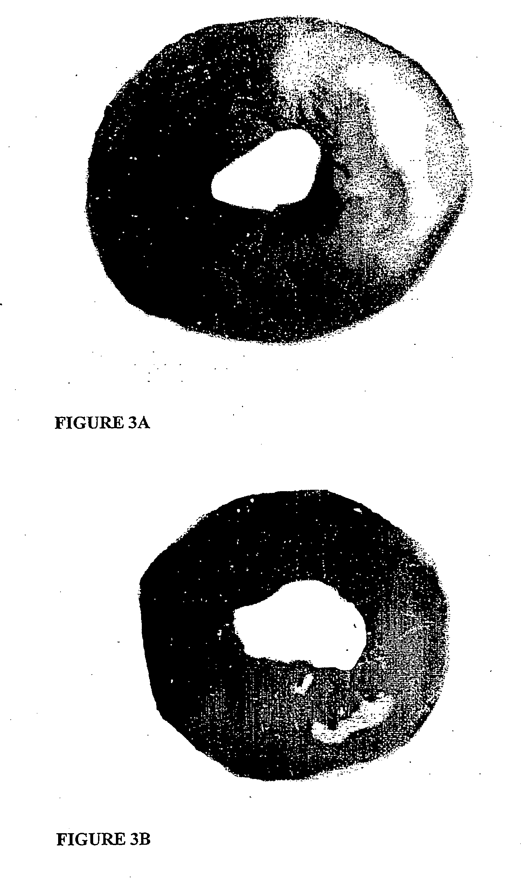

These results are also supported in the results shown in **Figures 3A-3B**, showing a slice of the heart of an animal from the control group (Fig. 3A), as well as a slice of the heart of an animal from the CBD-treated group (Fig. 3B). The white areas represent the infarct, which is significantly smaller in the CBD-treated group (Fig. 3B) than in the control group (Fig. 3A).

Hearts from each group were stained with hematoxylin, and eosin and Masson trichrome (**Figures 4A, 4B**, **4E-4G**). The inflammatory infiltrate was graded on a 1-3 scale by an expert pathologist based on the number of leukocytes infiltrating the border of the infarcted zone. The inflammatory response in the control animals was graded between 2-3, compared to a grade of 1+ or nullnil (0) in the CBD animals (Figures 4A and 4B, respectively), indicating the elimination of the inflammatory process in the CBD-treated animals. However, granulation tissue formation with early collagen deposition was similar in the treated and control and CBD-treated animals (Figures 4E and 4F, respectively). Further, Figure 4G is a higher magnification from control animals, showing replacement of necrotic tissue by early fibrovascular granulation tissue. Identical findings were seen in CBD-treated hearts (data not shown) (Masson trichrome x 200).

The results demonstrate that CBD compound has a significant *in vivo* cardioprotective effect against myocardial ischemia induced by LAD ligation, with reduction of infarct size, measured as percentage of the area at risk using TTC staining. This was accompanied by a significant increase in left ventricular function that is observed after coronary occlusion, as measured by echocardiogaphy. The reduction of the infarct size was associated with a qualitative reduction in inflammatory infiltration of the ischemic and necrotic myocardial zones. The CBD compound did not affect early collagen formation, as detected by Masson trichrome staining.

## Claims

1. Use of a cannabidiol (CBD) compound for the preparation of a pharmaceutical composition for treatment of at least one fundamental parameter affecting a vascular system selected from (a) the cardiovascular system; (b) the peripheral vascular system; or (c) a combination of (a) and (b), the fundamental parameter being selected from at least one of (i) dimension of heart scars; (ii) blood/plasma levels; (iii) atherosclerosis.

2. Use according to Claim 1, for the preparation of a composition for treatment of at least one fundamental parameter affecting the cardiovascular system.

3. Use according to Claim 1 or 2, wherein said treatment comprises reducing dimension of the heart scars.

4. Use according to any one of Claims 1 to 3, wherein said treatment comprises improvement in lipid blood/plasma levels.

5. Use according to Claim 4, wherein said improvement is selected from:
**(a)** reduction or prevention of elevation of a lipid level, wherein said lipid is selected from triglycerides, low density lipoprotein (LDL) or cholesterol;
**(b)** increase or prevention of decrease in HDL level or in HDL/LDL ratio.

6. Use according to any one of Claims 1 to 5, wherein said composition is for the treatment of at least one fundamental parameter, selected from blood and/or plasma lipid profile; atherosclerosis plaque load; dimension of heart scar.

7. Use according to Claim 1, wherein said pharmaceutical composition is for reducing atherosclerotic plaque load or preventing the build-up of atherosclerosis plaques.

8. Use according to any one of Claims 2 to 7, for the preparation of said pharmaceutical composition for treatment in combination with an invasive cardio-, coronary, or peripheral procedure for treating one or more of said fundamental parameters.

9. The use of any one of Claims 1 to 9, wherein said CBD compound comprises a general formula (I): 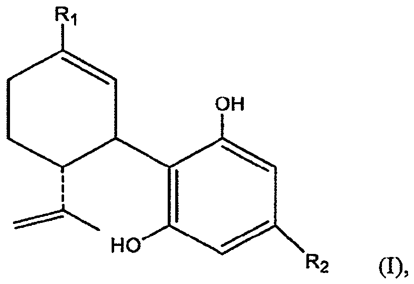 wherein
**R₁** is an alkyl; and
**R₂** is selected from: a straight or branched alkyl having 5 to 12 carbon atoms; an -OR₃ group, wherein **R₃** is a straight or branched alkyl having 5 to 9 carbon atoms or a straight or branched alkyl substituted at the terminal carbon atom by a phenyl group; or a -(CH₂)ₙ-O-alkyl group, wherein n is an integer from 1 to 7 and the alkyl group has 1 to 5 carbons.

10. Use according to Claim 9, wherein **R₁** is CH₃ and **R₂** is a straight alkyl having the formula -C₅H₁₁.

11. Use according to Claim 9 or 10, wherein said CBD is a cannabidiol of formula (II):

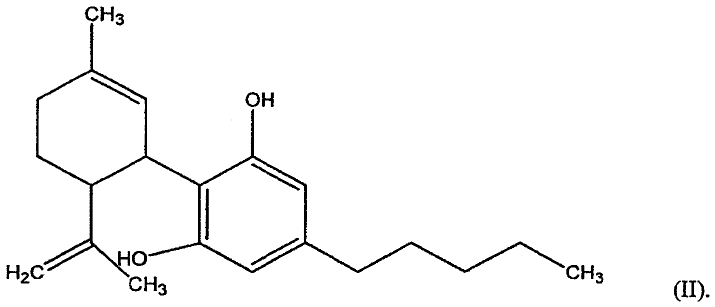

12. A biocompatible device comprising a CBD compound, the device being configured for deployment into a subject's vascular system selected from the cardiovascular system, the peripheral vascular system or both, such that upon deployment of the device in said subject's vascular system, said CBD compound is released from the device in an amount effective to treat or prevent at least one fundamental parameter affecting the vascular system.

13. A device according to Claim 12, comprising a radially expandable wire, stent or balloon, perforated tube, catheter, intravascular needle, or an ostial stent or balloon.

14. A device according to Claim 12 or 13, wherein said CBD compound is formulated into a pharmaceutical composition comprising a physiologically acceptable carrier permitting *in situ* release of the CBD compound, said release being at least at the area of insertion of the device in the vascular system, the device comprising said CBD compound by means of impregnation into a component of the device, coating or deposition onto a surface of the device, sequestering in holes, grooves, or pores of the device or sequestering within an inner space of the device.

## Patentansprüche

1. Verwendung einer Cannabidiol- (CBD-) -Verbindung für die Herstellung einer pharmazeutischen Zusammensetzung zur Behandlungwenigstens eines Grundparameters, der ein vaskuläres System beeinträchtigt, das aus (a) dem kardiovaskulären System; (b) dem peripheren vaskulären System oder (c) einer Kombination aus (a) und (b) ausgewählt ist, wobei der Grundparameter aus wenigstens einem von (i) einem Ausmaß von Herznarben; (ii) Blut/Plasma-Spiegeln; (iii) Atherosklerose ausgewählt ist.

2. Verwendung gemäß Anspruch 1 für die Herstellung einer Zusammensetzung zur Behandlung wenigstens eines Grundparameters, der das kardiovaskuläre System beeinträchtigt.

3. Verwendung gemäß Anspruch 1 oder 2, wobei die Behandlung eine Reduzierung des Ausmaßes der Herznarben umfasst.

4. Verwendung gemäß einem der Ansprüche 1 bis 3, wobei die Behandlung eine Verbesserung der Lipid-Blut/Plasma-Spiegel umfasst.

5. Verwendung gemäß Anspruch 4, wobei die Verbesserung ausgewählt ist aus:
(a) Reduzierung oder Prävention einer Erhöhung eines Lipidspiegels, wobei das Lipid aus Triglyceriden, Lipoprotein niederer Dichte (LDL) oder Cholesterin ausgewählt ist;
(b) Erhöhung oder Prävention einer Verringerung eines HDL-Spiegels oder eines HDL/LDL-Verhältnisses.

6. Verwendung gemäß einem der Ansprüche 1 bis 5, wobei die Zusammensetzung zur Behandlung wenigstens eines Grundparameters dient, der aus einem Blut- und/oder Plasma-Lipid-Profil, einer Atherosklerose-Plaque-Belastung; einem Ausmaß von Herznarben ausgewählt ist.

7. Verwendung gemäß Anspruch 1, wobei die pharmazeutische Zusammensetzung zum Reduzieren einer atherosklerotischen Plaque-Belastung oder zur Prävention des Aufbaus von Atherosklerose-Plaques dient.

8. Verwendung gemäß einem der Ansprüche 2 bis 7 für die Herstellung der pharmazeutischen Zusammensetzung zur Behandlung in Kombination mit einer invasiven Kardio-, Koronar-oder peripheren Prozedur zum Behandeln von einem oder mehreren der Grundparameter.

9. Verwendung gemäß einem der Ansprüche 1 bis 9, wobei die CBD-Verbindung eine allgemeine Formel (I) umfasst: 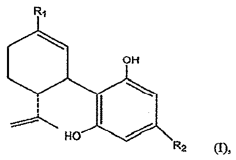 wobei
R₁ ein Alkyl ist und
R₂ ausgewählt ist aus: einem geradlinigen oder verzweigten Alkyl mit 5 bis 12 Kohlenstoffatomen; einer -OR₃-Gruppe, wobei R₃ ein geradliniges oder verzweigtes Alkyl mit 5 bis 9 Kohlenstoffatomen oder ein geradliniges oder verzweigtes Alkyl, das an dem terminalen Kohlenstoffatom durch eine Phenylgruppe ersetzt ist; oder einer -(CH₂)ₙ-O-Alkylgruppe, wobei n eine ganze Zahl von 1 bis 7 ist und die Alkylgruppe 1 bis 5 Kohlenstoffatome hat.

10. Verwendung gemäß Anspruch 9, wobei R₁ CH₃ ist und R₂ ein geradliniges Alkyl mit der Formel-C₅H₁₁ ist.

11. Verwendung gemäß Anspruch 9 oder 10, wobei das CBD ein Cannabidiol der Formel (II) ist:

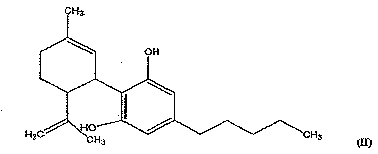

12. Biokompatible Vorrichtung, die eine CBD-Verbindung umfasst, wobei die Vorrichtung zur Ausbringung in ein vaskuläres System eines Subjekts, ausgewählt aus dem kardiovaskulären System, dem peripheren vaskulären System oder beiden, in der Weise ausgebildet ist, dass die CBD-Verbindung bei Ausbringung der Vorrichtung in dem vaskulären System des Subjekts aus der Vorrichtung in einer Menge abgegeben wird, die zur Behandlung oder Prävention wenigstens eines Grundparameters wirksam ist, der das vaskuläre System beeinträchtigt.

13. Vorrichtung gemäß Anspruch 12, umfassend einen radial expandierbaren Draht, Stent oder Ballon, eine perforierte Röhre, einen Katheter, eine intravaskuläre Nadel oder einen ostialen Stent oder Ballon.

14. Vorrichtung gemäß Anspruch 12 oder 13, wobei die CBD-Verbindung in eine pharmazeutische Zusammensetzung formuliert ist, die einen physiologisch akzeptablen Träger umfasst, der eine In-situ-Abgabe der CBD-Verbindung erlaubt, wobei die Abgabe wenigstens an dem Bereich der Einführung der Vorrichtung in das vaskuläre System erfolgt, wobei die Vorrichtung die CBD-Verbindung vermittels einer Imprägnierung in einen Bestandteil der Vorrichtung, einer Beschichtung oder Ablagerung auf eine Oberfläche der Vorrichtung, eine Sequestrierung in Löchern, Rillen oder Poren der Vorrichtung oder Sequestrierung in einem inneren Raum der Vorrichtung umfasst.

## Revendications

1. Utilisation d'un composé de cannabidiol (CBD) destiné à la préparation d'une composition pharmaceutique destinée au traitement d'au moins un paramètre fondamental affectant un système vasculaire sélectionné parmi (a) le système cardio-vasculaire ; (b) le système vasculaire périphérique ; ou une combinaison de (a) et (b), le paramètre fondamental étant sélectionné parmi au moins un des facteurs de (i) dimension des cicatrices du coeur ; (ii) taux sanguins/plasmatiques ; (iii) athérosclérose.

2. Utilisation selon la revendication 1, destinée à la préparation d'un traitement d'au moins un paramètre fondamental affectant le système cardiovasculaire.

3. Utilisation selon les revendications 1 ou 2, dans laquelle ledit traitement comprend la réduction de la dimension des cicatrices du coeur.

4. Utilisation selon une quelconque des revendications 1 ou 2, dans laquelle ledit traitement comprend l'amélioration des taux sanguins/plasmatiques de lipides.

5. Utilisation selon la revendication 4, dans laquelle ladite amélioration est sélectionnée à partir de:
(a) la réduction ou la prévention de l'élévation d'un taux de lipides, dans laquelle ledit lipide est sélectionné parmi les triglycérides, la lipoprotéine à basse densité (LDL) ou le cholestérol ;
(b) l'augmentation ou la prévention de la réduction du taux HDL ou du taux HDL/LDL.

6. Utilisation selon une quelconque des revendications 1 à 5, dans laquelle ladite composition est destinée au traitement d'au moins un des paramètres fondamentaux, sélectionné parmi le profil de lipides sanguins et/ou plasmatiques ; la charge de plaque athérosclérotique ; la dimension de la cicatrice du coeur.

7. Utilisation selon la revendication 1, dans laquelle ladite composition pharmaceutique est destinée à la réduction de la charge de plaque athérosclérotique ou à la prévention de la formation de plaques athérosclérotiques.

8. Utilisation selon une quelconque des revendications 2 à 7, destinée à la préparation de ladite composition pharmaceutique destinée au traitement en combinaison avec une procédure invasive cardiologique, coronaire ou périphérique destinée au traitement d'un ou plusieurs des paramètres fondamentaux.

9. Utilisation selon une quelconque des revendications 1 à 9, dans laquelle ledit composé CBD comprend une formule générale (I) : 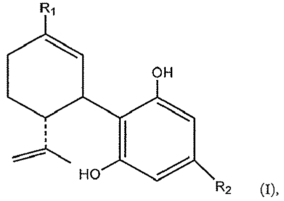 dans laquelle
R₁ est un alkyle ; et
R₂ est sélectionné parmi: un alkyle droit ou ramifié possédant 5 à 12 atomes de carbone ; ou un groupe OR₃, dans lequel R₃ est un alkyle droit ou ramifié possédant 5 à 9 atomes de carbone ou un alkyle droit ou ramifié substitué sur l'atome de carbone terminal par un groupe de phényle ; ou un groupe de (CH₂)ₙ-O-alkyle, dans lequel n est un nombre entier de 1 à 7 et le groupe d'alkyle possède 1 à 5 atomes de carbone.

10. Utilisation selon la revendication 9, dans laquelle R₁ est du CH₃ et R₂ est un alkyle droit possédant la formule -C₅H₁₁.

11. Utilisation selon les revendications 9 ou 10, dans laquelle ledit CBD est un cannabidiol de la formule (II) :

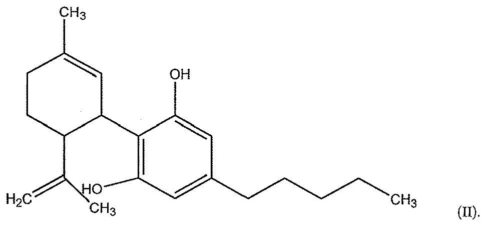

12. Dispositif biocompatible comprenant un composé de CBD, le dispositif étant configuré pour le déploiement dans un système vasculaire d'un sujet, sélectionné parmi le système cardio-vasculaire, le système vasculaire périphérique ou les deux, de sorte que lors du déploiement dans ledit système vasculaire d'un sujet, ledit composé CBD est relâché à partir du dispositif dans une quantité efficace pour traiter ou pour prévenir au moins un paramètre fondamental affectant le système vasculaire.

13. Dispositif selon la revendication 12, comprenant un fil métallique, une rame ou un ballon extensibles radialement, un tube perforé, un cathéter, une aiguille intravasculaire, ou une rame ou un ballon ostiaux.

14. Dispositif selon les revendications 12 ou 13, dans lequel ledit composé CBD est formulé en une composition pharmaceutique comprenant un support acceptable physiologiquement permettant le relâchement in situ du composant CBD, ledit relâchement étant au moins sur la zone d'insertion du dispositif dans le système vasculaire, le dispositif comprenant ledit composé CBD au moyen de l'imprégnation dans un composé du dispositif, du revêtement ou de la déposition sur une surface of du dispositif, de la séquestration dans des trous, des rainures ou des pores du dispositif ou la séquestration dans un espace interne du dispositif.
